# EUROPÄISCHE PATENTSCHRIFT

(11) **EP 1 374 854 B1**
(45) Veröffentlichungstag und Bekanntmachung des Hinweises auf die Patenterteilung: **26.07.2006**
(21) Anmeldenummer: 03011965.5
(22) Anmeldetag: 28.05.2003
(51) Int. Cl.: A61K 9/00, A61K 9/20, A61K 45/06

(54) **Pharmazeutische Zubereitung mit retardierender Wirkstoffreisetzung, Verfahren zu ihrer Herstellung und Verwendung**
Delayed release pharmaceutical composition, method of production and use of said composition
Composition pharmaceutique à libération prolongée, procédé de production et utilisation de celle-ci

(30) Priorität: 21.06.2002 DE 10227914
(43) Veröffentlichungstag der Anmeldung: 02.01.2004
(73) Patentinhaber: Heraeus Kulzer GmbH, 63450 Hanau (DE)
(72) Erfinder: Vogt, Sebastian, Dr., 99084 Erfurt (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- EP-A- 0 611 571
- US-A1- 2002 004 071
- WICHELHAUS, T. A.; ET AL.: "Elution characteristics of vancomycin, teicoplanin, gentamicin and clindamycin from calcium sulfate beads." JOURNAL OF ANTIMICROBIAL CHEMOTHERAPY, Bd. 48, Nr. 1, 2001, Seiten 117-119, XP002256357 GB

## Beschreibung

Die vorliegende Erfindung betrifft eine pharmazeutische Zubereitung mit retardierender Wirkstofffreisetzung als resorbierbare und auch als nichtresorbierbare Implantate für die Human- und Veterinärmedizin zur Behandlung schwerer, lokaler bakterieller Infektionen im Hart- und im Weichgewebe. Insbesondere soll die pharmazeutische Zubereitung bei der Therapie von bakteriellen Infektionen Anwendung finden, die auf Grund von Resistenz-Erscheinungen einer einfachen lokalen Antibiotika-Behandlung mit nur einem Antibiotikum nicht mehr zugänglich sind. Die Erfindung betrifft des weiteren ein Verfahren zur Herstellung und die Verwendung der Zubereitung.

Die Behandlung lokaler mikrobieller Infektionen des Weich- und Hartgewebes in der Human- und Veterinärmedizin erfordert hohe lokale Antibiotika-Konzentrationen im infizierten Gewebebereich. Es ist seit langem bekannt, dass eine systemische Anwendung von Antibiotika mit einer Reihe von Problemen behaftet ist. Bei der systemischen Anwendung ist es oft erforderlich sehr hohe Antibiotika-Dosen einzusetzen, damit im infizierten Gewebe antimikrobiell wirksame Antibiotika-Konzentrationen erzielt werden. Dadurch kann es insbesondere bei den Aminoglycosid-Antibiotika auf Grund ihrer Nephro- und Ototoxizität zu schwerwiegenden Schädigungen des Organismus kommen. Daher lag der Gedanke nahe, Antibiotika in lokal applizierbaren Freisetzungssystemen einzusetzen, beziehungsweise sie in geeignete Depotformen zu überführen. Weiterhin ist es sinnvoll, dass die lokal applizierbaren Freisetzungssysteme in den ersten Stunden eine hohe Wirkstofffreisetzung zeigen und anschließend über einen Zeitraum von mehreren Tagen kontinuierlich geringe Wirkstoffmengen abgeben, damit eine weitgehende Abtötung der bakteriellen Erreger erreicht wird.

Teicoplanin ist ein Glykopeptidantibiotikum, dass gegenüber Gram-positiven bakteriellen Keimen wirksam ist. Es inhibiert die Murein-Synthese und damit die Quervernetzung der bakteriellen Zellwände. Teicoplanin hat insbesondere die Vorteile, dass es im Vergleich zu β-Lactam-Antibiotika wesentlich lagerungsstabiler ist und dass es bei Patienten mit Penicillin-Allergie eingesetzt werden kann. Aminoglykosid-Antibiotika, wie Gentamicin und Kanamycin, und auch Clindamycin beeinträchtigen die bakterielle Proteinsynthese und haben dadurch bei vielen Gram-positiven Bakterien, anaeroben Bakterien und zum Teil auch bei Gram-negativen Bakterien eine bakterizide Wirkung. Fluorchinolon-Antibiotika, wie Ciprofloxacin und Moxifloxacin, stellen Breitband-Antibiotika dar und wirken als Topoisomerase-Hemmer und als Gyrase-Hemmer gegen eine Vielzahl von Gram-positiven Bakterien. Bei der Behandlung von Problemkeimen ist es daher sinnvoll, zwei Antibiotika miteinander zu kombinieren, die unterschiedliche Angriffsorte im bakteriellen Stoffwechsel haben. Dadurch erhöht sich die Wahrscheinlichkeit eine wirksamen Bekämpfung dieser Problemkeime.

Im EP 0 611 571 wird ein lokal applizierbares Arzneimittel mit verzögerter Freisetzung in Form eines Trockenproduktes oder einer Suspension, davon in einem inerten flüssigen Träger, enthaltend als schwer wasserlösliches Produkt, die Kombination aus Teicoplanin und einem weiteren Arzneimittel mit basischen Charakter, offengelegt. Als basische Arzneimittel werden die Aminoglykoside Gentamicin, Netilmicin und Tobramicin genannt. Bei dieser Offenlegungsschrift werden schwerlösliche Reaktionsprodukte aus Teicoplanin mit Gentamicin, Teicoplanin mit Netilmicin sowie Teicoplanin mit Tobramycin beansprucht, die als lokal applizierbare Arzneimittel in Form von Trockenprodukten oder einer Suspension eingesetzt werden.

Im US 2002/0004071 A1 wird eine halbfeste Zusammensetzung enthaltend Hilfstoffe und Teicoplanin, Vancomycin HCl, Amikacinsulfat oder Gemische davon offengelegt.

Es wurden bisher keine Veröffentlichungen bekannt, die pharmazeutische Zubereitungen beschreiben, die aus Gemischen von pulverförmigem Teicoplanin und pulverförmigen, in Wasser löslichen Salzen des Gentamicins, des Clindamycins, des Gentamicins, des Clindamycins, des Kanamycins, des Amikacins, des Tobramycins, des Vancomycins, des Moxifloxacins und des Ciprofloxacins und geeigneter anorganischer und/oder organischer Hilfsstoffe bestehen und die im wäßrigem Milieu eine verzögerte Wirkstofffreisetzung zeigen.

Der Erfindung liegt die Aufgabe zu Grunde, eine pharmazeutische Zubereitung zu entwickeln, die Teicoplanin und andere Antibiotika enthält und im wäßrigen Milieu, wie unter physiologischen Bedingungen, die Antibiotika über einen Zeitraum von mehreren Tagen verzögert freisetzt.

Die Aufgabe wird erfindungsgemäß durch die Merkmale der unabhängigen Ansprüche gelöst. Vorteilhafte Ausgestaltungen sind in den Unteransprüchen angegeben.

Der Erfindung liegt der überraschende Befund zu Grunde, dass Gemische aus pulverförmigem Teicoplanin und pulverförmigen, in Wasser löslichen Salzen des Gentamicins, des Clindamycins, des Gentamicins, des Clindamycins, des Kanamycins, des Amikacins, des Tobramycins, des Vancomycins, des Moxifloxacins und des Ciprofloxacins in Gegenwart geeigneter anorganischer Hilfsstoffe und/oder organischer Hilfsstoffe eine verzögerte Wirkstofffreisetzung im wäßrigen Milieu zeigen. Es ist überraschend, im Gegensatz zum EP 0 611 571, bei dem in Wasser gering lösliche Addukte des Teicoplanins und anderer Antibiotika als Arzneimittel verwendet werden, dass bei der erfindungsgemäßen pharmazeutischen Zubereitung eine Synthese von schwerlöslichen Teicoplanin-Antibiotika-Addukten nicht notwendig ist und trotzdem eine verzögerte Antibiotika-Freisetzung der erfindungsgemäßen pharmazeutischen Zubereitung im wäßrigen Milieu gefunden wird. Die pharmazeutische Zubereitung ist in pharmazeutisch üblicher Weise herstellbar, ohne dass jedoch in Wasser gering lösliche Antibiotika-Salze in aufwendiger Synthese, wie bei EP 0 611 571, hergestellt werden müssen. Weiterhin ist die pharmazeutische Zubereitung für mehrere Kombinationen aus Teicoplanin und anderen Antibiotika geeignet. Die pharmazeutische Zubereitung ist mit unterschiedlichsten tablettierbaren Hilfsstoffen realisierbar.

Es ist erfindungsgemäß bevorzugt, dass als anorganische Hilfsstoffe Calciumcarbonat, Calciumsulfat-Dihydrat, Tricalciumphosphat und Hydroxylaptit verwendet werden.

Weiterhin ist erfindungsgemäß vorteilhaft, dass als organische Hilfsstoffe Polyester der Milchsäure, der Glykolsäure, der 5-Hydroxyvaleriansäure und der 6-Hydroxycapronsäure sowie deren Copolymere als organische Hilfsstoffe verwendet werden.

Bevorzugt ist es, dass die Gemische durch Pressen, durch Strangpressen, durch Spinnen und durch Granulieren zu Tabletten, zu Formkörpern, zu Fäden und zu Granulaten geformt werden.

Es ist des weiteren vorteilhaft, dass eine Kombination aus polymerisierbaren Methacrylsäureestern und Gemischen, die aus pulverförmigem Teicoplanin und pulverförmigen, in Wasser löslichen Salzen des Gentamicins, des Clindamycins, des Gentamicins, des Clindamycins, des Kanamycins, des Amikacins, des Tobramycins, des Vancomycins, des Moxifloxacins und des Ciprofloxacins gebildet ist, zu einem Formkörpern polymerisiert ist. Im Sinne der Erfindung sind dabei insbesondere Kugeln oder walzenähnliche Körper, die aus polymeren Methacrylsäureestern bestehen, welche die erfindungsgemäßen Gemische aus pulverförmigen Teicoplanin und mindestens einer pulverförmigen, wasserlöslichen Salzform des Gentamicins, des Clindamycins, des Kanamycins, des Amikacins, des Tobramycins, des Vancomycins, des Moxifloxacins und Ciprofloxacins enthalten, und als implantierbare Wirkstoffträger, analog zu den Septopal®-Ketten, zur lokalen Infektionsbekämfung eingesetzt werden. Es ist außerdem im Sinne der Erfindung, dass die erfindungsgemäßen Gemische aus pulverförmigem Teicoplanin und mindestens einer pulverförmigen, wasserlöslichen Salzform des Gentamicins, des Clindamycins, des Kanamycins, des Amikacins, des Tobramycins, des Vancomycins, des Moxifloxacins und Ciprofloxacins in Knochenzementen enthalten sein können. Unter dem Begriff der Formkörper werden auch die ausgehärteten Knochenzemente verstanden.

Es ist erfindungsgemäß, dass die Gemische, die aus pulverförmigem Teicoplanin und pulverförmigen, in Wasser löslichen Salzen des Gentamicins, des Clindamycins, des Gentamicins, des Clindamycins, des Kanamycins, des Amikacins, des Tobramycins, des Vancomycins, des Moxifloxacins und des Ciprofloxacins gebildet sind, anorganischen Calciumphosphat-Knochenzementen vor ihrer Härtung beigemischt werden. Im Sinne der Erfindung ist ebenfalls die Verwendung der Gemische in selbsthärtenden Calciumsulfat-Gemischen zur Auffüllung von Knochendefekten.

Es ist zweckmäßig, dass die Gemische Bestandteil von resorbierbaren und von nichtresorbierbaren Beschichtungen sind, die auf nichtmetallische und metallische Implantaten aufgebracht werden.

Erfindungsgemäß werden die pharmazeutischen Zubereitungen in Form von Tabletten, Formkörpern, Fäden und Granulaten als permanente Implantate und als temporäre Implantate verwendet.

Die Erfindung soll nachfolgend durch die Beispiele 1 bis 3 näher erläutert werden.

### Beispiel 1

Es wird ein Gemisch aus 500,0 mg Calciumsulfat-Dihydrat (Fluka), 125,0 mg Poly-L-lactid (M - 10000 g/mol), 18,7 mg Gentamicinsulfat (AK 628) und 18,7 mg Teicoplanin vermahlen. Jeweils 200 mg dieses Gemischs werden mit einer Presse bei einem Druck von 5000 kg (5 Tonnen) innerhalb von zwei Minuten zu scheibenförmigen Formkörpern, mit einem Durchmesser von 13 mm, gepresst.

### Beispiel 2

Es wird ein Gemisch aus 500,0 mg Calciumsulfat-Dihydrat (Fluka), 125,0 mg Poly-L-lactid (M - 10000 g/mol), 18,7 mg Clindamycinhydrochlorid und 18,7 mg Teicoplanin vermahlen. Jeweils 200 mg dieses Gemischs werden mit einer Presse bei einem Druck von 5000 kg (5 Tonnen) innerhalb von zwei Minuten zu scheibenförmigen Formkörpern, mit einem Durchmesser von 13 mm, gepresst.

### Beispiel 3

Es wird ein Gemisch aus 1000,0 mg Calciumsulfat-Dihydrat (Fluka), 250,0 mg Poly-L-lactid (M - 10000 g/mol), 18,7 mg Kanamycinsulfat (Fluka) und 18,7 mg Teicoplanin vermahlen. Jeweils 200 mg dieses Gemischs werden mit einer Presse bei einem Druck von 5000 kg (5 Tonnen) innerhalb von zwei Minuten zu scheibenförmigen Formkörpern, mit einem Durchmesser von 13 mm, gepresst.

### Antibiotika-Freisetzungsversuche

Die in den Beispielen 1-3 hergestellten Formkörper wurden in Sörensen-Puffer pH 7,4 eingebracht und in diesem bei 37 °C über einen Zeitraum von 12 Tagen gelagert. Die Probennahme erfolgte täglich, wobei das Freisetzungsmedium ausgetauscht wurde. Die Antibiotika-Freisetzung aus den Formkörpern wurde mit einem Agardiffusionstest unter Verwendung von Bacillus subtilis ATCC 6633 als Testkeim verfolgt. Es wurden die Hemmhofdurchmesser mit Hilfe eines Scanners und einer speziellen Auswertungssoftware ermittelt. Die Ergebnisse sind in der Tabelle dargestellt.

**Tab.: Ergebnisse des mikrobiellen Agar-Diffusions-Tests zur Erfassung der Antibiotika-Freisetzung aus Probekörpern der Beispiele 1-3 in Abhängigkeit von der Lagerungszeit der Probekörper im Sörensen-Puffer bei 37°C.**

| Zeit [d] | Beispiel 1 | | Beispiel 2 | | Beispiel 3 | |
|---|---|---|---|---|---|---|
| | Verdünnung | Hemmhofdurchmesser [mm] | Verdünnung | Hemmhofdurchmesser [mm] | Verdünnung | Hemmhofdurchmesser [mm] |
| 1 | 1:100 | 22,40 | 1:100 | 20,00 | 1:70 | 20,35 |
| 2 | 1:15 | 20,85 | 1:30 | 20,80 | 1:5 | 21,15 |
| 3 | 1:3 | 20,28 | 1:14 | 19,80 | unverdünnt | 21,45 |
| 6 | unverdünnt | 18,25 | unverdünnt | 21,55 | unverdünnt | 14,20 |
| 9 | unverdünnt | 15,63 | unverdünnt | 18,35 | unverdünnt | nicht ermittelt |
| 12 | unverdünnt | 17,70 | unverdünnt | 21,30 | unverdünnt | 15,25 |

## Patentansprüche

1. Pharmazeutische Zubereitung mit retardierender Wirkstofffreisetzung, **dadurch gekennzeichnet, dass** sie Gemische aus pulverförmigem Teicoplanin und mindestens einer pulverförmigen, wasserlöslichen Salzform des Gentamicins, des Clindamycins, des Kanamycins, des Amikacins, des Tobramycins, des Vancomycins, des Moxifloxacins und des Ciprofloxacins und eines anorganischen und/oder organischen Hilfsstoffes enthält.

2. Pharmazeutische Zubereitung mit retardierender Wirkstofffreisetzung nach Anspruch 1, **dadurch gekennzeichnet, dass** sie als anorganische Hilfsstoffe Calciumcarbonat, Calciumsulfat-Dihydrat, Tricalciumphosphat und/oder Hydroxylapatit enthält.

3. Pharmazeutische Zubereitung mit retardierender Wirkstofffreisetzung nach Anspruch 1 oder 2, **dadurch gekennzeichnet, dass** sie als organische Hilfsstoffe Polyester der Milchsäure, der Glykolsäure, der 5-Hydroxyvaleriansäure und der 6-Hydroxycapronsäure sowie deren Copolymere als organische Hilfsstoffe enthält.

4. Pharmazeutische Zubereitung mit retardierender Wirkstofffreisetzung nach einem der Ansprüche 1 bis 3, **dadurch gekennzeichnet, dass** die Gemische die Form von Tabletten, Formkörpern, Fäden und Granulaten aufweisen.

5. Pharmazeutische Zubereitung mit retardierender Wirkstofffreisetzung nach Anspruch 1, **dadurch gekennzeichnet, dass** eine Kombination aus polymerisierbaren Methacrylsäureestem und Gemischen, die aus pulverförmigem Teicoplanin und mindestens einer pulverförmigen, wasserlöslichen Salzform des Gentamicins, des Clindamycins, des Kanamycins, des Amikacins, des Tobramycins, des Vancomycins, des Moxifloxacins und des Ciprofloxacins gebildet und zu einem Formkörper polymerisiert ist.

6. Pharmazeutische Zubereitung mit retardierender Wirkstofffreisetzung nach einem der Ansprüche 1 bis 4, **dadurch gekennzeichnet, dass** die Gemische Bestandteil von resorbierbaren und/oder von nichtresorbierbaren Beschichtungen sind, die auf nichtmetallischen und metallischen Implantaten aufgebracht sind.

7. Pharmazeutische Zubereitung mit retardierender Wirkstofffreisetzung nach Anspruch 1, **dadurch gekennzeichnet, dass** anorganischen Calciumphosphat-Knochenzementen und Gipsgemischen vor ihrer Härtung Gemische, die aus pulverförmigem Teicoplanin und mindestens einer pulverförmigen, wasserlöslichen Salzform des Gentamicins, des Clindamycins, des Kanamycins, des Amikacins, des Tobramycins, des Vancomycins, des Moxifloxacins und/oder des Ciprofloxacins gebildet sind, beigefügt werden.

8. Verwendung einer pharmazeutischen Zubereitung mit retardierender Wirkstofffreisetzung nach einem der Ansprüche 1 bis 5 in Form von Tabletten, Formkörpern, Fäden und Granulaten als permanente oder temporäre Implantate.

## Claims

1. Pharmaceutical formulation having retarded active substance release, **characterized in that** it contains mixtures of pulverulent teicoplanin and at least one pulverulent, water-soluble salt form of gentamicin, of clindamycin, of kanamycin, of amikacin, of tobramycin, of vancomycin, of moxifloxacin and of ciprofloxacin and of an inorganic and/or organic excipient.

2. Pharmaceutical formulation having retarded active substance release according to Claim 1, **characterized in that** it contains calcium carbonate, calcium sulphate dihydrate, tricalcium phosphate and/or hydroxylapatite as inorganic excipients.

3. Pharmaceutical formulation having retarded active substance release according to Claim 1 or 2, **characterized in that** it contains polyesters of lactic acid, of glycolic acid, of 5-hydroxyvaleriac acid and 6-hydroxycapronic acid and copolymers thereof as organic excipients.

4. Pharmaceutical formulation having retarded active substance release according to any of Claims 1 to 3, **characterized in that** the mixtures have the form of tablets, mouldings, filaments and granules.

5. Pharmaceutical formulation having retarded active substance release according to Claim 1, **characterized in that** a combination is formed from polymerizable methacrylic esters and mixtures which consist of pulverulent teicoplanin and at least one pulverulent, water-soluble salt form of gentamicin, of clindamycin, of kanamycin, of amikacin, of tobramycin, of vancomycin, of moxifloxacin and of ciprofloxacin and is polymerised to give a moulding.

6. Pharmaceutical formulation having retarded active substance release according to any of Claims 1 to 4, **characterized in that** the mixtures are a constituent of absorbable and/or of non-absorbable coatings which are applied to non metallic and metallic implants.

7. Pharmaceutical formulation having retarded active substance release according to Claim 1, **characterized in that** mixtures which are formed from pulverulent teicoplanin and at least one pulverulent, water-soluble salt form of gentamicin, of clindamycin, of kanamycin, of amikacin, of tobramycin, of vancomycin, of moxifloxacin and/or of ciprofloxacin are added to inorganic calcium phosphate bone cements and gypsum mixtures before their hardening.

8. Use of a pharmaceutical formulation having retarded active substance release according to any of Claims 1 to 5 in the form of tablets, mouldings, filaments and granules as permanent or temporary implants.

## Revendications

1. Composition pharmaceutique à libération retardée de la substance active, **caractérisée en ce qu'**elle contient des mélanges de teicoplanine pulvérulente et d'au moins une forme de sel soluble dans l'eau pulvérulente de la gentamycine, de la clindamycine, de la kanamycine, de l'amikacine, de la tobramycine, de la vancomycine, de la moxifloxacine et de la ciprofloxacine et d'un produit auxiliaire inorganique et/ou organique.

2. Composition pharmaceutique à libération retardée de la substance active selon la revendication 1, **caractérisée en ce qu'**elle contient comme produits auxiliaires inorganiques du carbonate de calcium, du sulfate de calcium dihydraté, du phosphate tricalcique et/ou de l'hydroxyapatite.

3. Composition pharmaceutique à libération retardée de la substance active selon la revendication 1 ou 2, **caractérisée en ce qu'**elle contient comme produits auxiliaires organiques des polyesters de l'acide lactique, de l'acide glycolique, de l'acide 5-hydroxyvalérique et de l'acide 6-hydroxycaproïque et leurs copolymères comme produits auxiliaires organiques.

4. Composition pharmaceutique à libération retardée de la substance active selon l'une des revendications 1 à 3, **caractérisée en ce que** les mélanges présentent la forme de comprimés, de corps moulés, de fils et de granulés.

5. Composition pharmaceutique à libération retardée de la substance active selon la revendication 1, **caractérisée en ce qu'**une combinaison d'esters d'acide méthacrylique polymérisables et de mélanges formés de teicoplanine pulvérulente et d'au moins une forme de sel soluble dans l'eau pulvérulente de la gentamycine, de la clindamycine, de la kanamycine, de l'amikacine, de la tobramycine, de la vancomycine, de la moxifloxacine et de la ciprofloxacine est polymérisée en un corps moulé.

6. Composition pharmaceutique à libération retardée de la substance active selon l'une des revendications 1 à 4, **caractérisée en ce que** les mélanges sont un constituant de revêtements résorbables et/ou non résorbables qui sont appliqués sur des implants non métalliques et métalliques.

7. Composition pharmaceutique à libération retardée de la substance active selon la revendication 1, **caractérisée en ce que** des mélanges formés de teicoplanine pulvérulente et d'au moins une forme de sel soluble dans l'eau pulvérulente de la gentamycine, de la clindamycine, de la kanamycine, de l'amikacine, de la tobramycine, de la vancomycine, de la moxifloxacine et/ou de la ciprofloxacine sont ajoutés à des ciments osseux inorganiques à base de phosphate de calcium et des mélanges de gypse avant leur durcissement.

8. Utilisation d'une composition pharmaceutique à libération retardée de la substance active selon l'une des revendications 1 à 5 sous forme de comprimés, de corps moulés, de fils et de granulés comme implants permanents ou temporaires.
